# EUROPEAN PATENT SPECIFICATION

(11) **EP 1 144 592 B1**
(45) Date of publication and mention of the grant of the patent: **29.10.2008**
(21) Application number: 00901189.1
(22) Date of filing: 21.01.2000
(51) Int. Cl.: C12N 5/00, A61L 15/00, A61L 27/00, C08L 5/00

(54) **SUBSTRATE FOR CELL GROWTH**
TRÄGER ZUM ZELLWACHSTUM
SUBSTRAT POUR CROISSANCE CELLULAIRE

(30) Priority: 21.01.1999 GB 9901272; 17.02.1999 GB 9903561
(43) Date of publication of application: 17.10.2001
(73) Proprietor: Advanced Medical Solutions Limited, Winsford, Cheshire CW7 3PD (GB)
(72) Inventor: HAMILTON, Douglas, William, Northwich, Cheshire CW8 2AJ (GB); IVES, Christopher, Louis, Nantwich, Cheshire CW5 8QA (GB); MIDDLETON, Ian, Philip, Chester CH3 5RR (GB); ROSSETTO, Chiara, Warrington, Cheshire WA5 1EW (GB)
(74) Representative: Atkinson, Peter Birch
(86) International application number: PCT/GB2000/000145
(87) International publication number: WO 2000/049135

(56) References cited:
- WO-A-96/10106
- WO-A-98/12228
- DATABASE WPI Section Ch, Week 199005 Derwent Publications Ltd., London, GB; Class A96, AN 1990-032925 XP002147316 & JP 01 309682 A (SANYO CHEM IND LTD), 14 December 1989 (1989-12-14)

## Description

The present invention relates to substrates for use in cell growth and to methods of producing such substrates. The invention relates more particularly to substrates having cell adhesion promoting activity which may be used in various cell growth applications, e.g. wound healing and tissue engineering. The invention also relates to methods of preparing such substrates and their use in various cell growth applications.

All eukaryotic, mammalian cells are substrate dependent in that they need to be attached to a surface in order to be able to grow, or secrete or divide. The phenotype that cells express is partly determined by their interaction with the substrate to which they are attached. The substrate to which mammalian cells are attached is collagen. All body soft (excluding blood) and hard tissues are made up of cells attached to a framework of collagen. Collagen is a protein that forms fibres and the fibres form matrices, these matrices may form any configuration from random to aligned.

The collagen fibres are themselves made up of fibrils so a collagen fibre resembles a cable of aligned fibrils. The chemistry of the collagen fibril varies according to the tissue type and a range of collagens have been identified.

Substrates for tissue augmentation or to act as carriers for cultured cell transfer in wound therapy are usually collagen based. In this situation, the collagen substrate usually has to be specific to the type of cell growth required and the phenotype and status (secretory, replicatory) grown on the substrate may not turn out to be as required.

WO-A-9812228 discloses modified alginates which comprise at least one alginate chain to which is covalently bonded at least one molecule useful for cell interaction, e.g. a cell adhesion protein. The materials are useful for providing polymeric matrices for tissue engineering applications for bone or soft tissue replacement.

US-A-5 610 148 (R.Brown) entitled "Macroscopically Orientated Cell Adhesion Protein" describes the production of a fibre comprised of fibrils of a cell adhesion protein selected from fibronectin (Fn), vitronectin and von Willebrand protein that has been denatured and the polymer chains then aligned by unidirectional shear allowing aggregation and precipitation. These fibres are of a fibular construction not dissimilar in some respects to collagen. Cells seeded onto the fibres demonstrate directional cell growth as a result of the longitudinal orientation of the cell adhesion binding site. However such fibre structures require a high concentration of fibronectin or fibrinogen/fibronectin, are somewhat complicated to produce and are of relatively low strength.

It is an object of the present invention obviate or mitigate the above mentioned disadvantages.

According to the present Invention there is provided a substrate for cell growth comprising a polysaccharide and a cell adhesion protein provided at the surface of the substrate and covalently associated therewith by hydrogen bonding van der Waals forces or physical entrapment.

Substrates in accordance with the invention have the advantage (over substrates comprised of fibrils of fibronectin or other cell adhesion protein) of being of higher strength than a substrate comprised substantially of 100% protein and are also easier to manufacture. The substrates of the invention may be used in a wide range of cell growth applications, e.g. wound repair, tissue repair or augmentation, or for the growth of cells in routine cell culture in vitro, in large scale cell culture, bioreactors or organ culture.

In the substrates of the invention, the orientation of the cell adhesion protein is not necessarily significant and guidance of the cells during growth thereof is achieved by the physical form of the substrate. Thus, for example, in the case of a fibre (see below) cell growth may occur along and/or around the fibre as determined by the presence of the cell adhesion protein. We do not however preclude the possibility of the cell adhesion protein having at least some degree of alignment.

The cell adhesion protein preferably incorporates the RGD (Arginine, Glycuse, Aspartic acid) binding site. It is particularly preferred that the cell adhesion protein is fibronectin, vitronectin or von Willebrand protein or a fragment of such proteins incorporating this RGD binding site.

The preferred cell adhesion protein is fibronectin which may be used in the form routinely isolated from blood plasma, e.g. by cryoprecipitation. The fibronectin may contain fibrinogen and albumin.

The polysaccharide and the cell adhesion protein may be uniformly distributed throughout the substrate so that the cell adhesion protein is present at the surface as a result of this distribution.

The substrate may comprise a polysaccharide basal layer having a surface layer of a cell adhesion protein.

The polysaccharide basal layer will for preference have a thickness of at least 60%, more preferably at least 80% and ideally at least 90% of the combined depth of the basal layer and cell adhesion protein layer.

The cell adhesion protein provided as a surface layer for the polysaccharide basal layer may be an integral layer or may be a surface absorbed molecular layer. The surface layer of the cell adhesion protein may, depending on the method by which it is produced, be only several molecules thick or may be of somewhat greater thickness so as to form a discrete outer layer. Thus, the protein layer may be anything from 3-5 molecules "deep" in the case of surface adsorption to, say, 20µm (e.g. 1-20 µm) when formed as a "coating". This protein layer may be an essentially amorphous network, have some crystallinity or even little or no fibril structure. The protein layer may be stabilised and attached to the basal (polysaccharide) layer by hydrogen bonding, van der Waals forces for physical entrapment. The degree of stability of the protein layer can be used as a mechanism to drive certain cell responses. Thus the substrate may be "tailored" to ensure growth of a particular cell type and/or to provide a known degree of cell growth in a predetermined time.

The polysaccharide layer will for preference comprise at least 50%, more preferably at least 60%, even more preferably at least 80% and ideally at least 90% polysaccharide. The cell adhesion protein layer will preferably comprise at least say more preferably at least 60% even more preferably at least 80% and ideally at least 90% of cell adhesion protein.

The cell adhesion protein layer may incorporate proteins other than cell adhesion proteins.

Cell growth substrates in accordance with the invention may incorporate, e.g. in the polysaccharide layer, an active agent for delivery during the cell growth application. This agent may, for example, be deliverable by diffusion and might for example be a drug. Further examples of active agents include growth factors, chemotactic agents etc. The active agents may be free or encapsulated, for example in lipid type droplets. The active agent may be disposed continuously or discontinuously along, across and/or around the cell growth substrate and may be provided in different amounts at different regions of the substrate so as to establish a concentration gradient.

Substrates in accordance with the invention may be produced by a number of methods. In one such method, a solution containing dissolved polysaccharide and cell adhesion protein (the solution containing less of the protein than the polysaccharide) is extruded into a coagulation bath. We believe that, in such a method, there is preferential deposition of the cell adhesion. The coagulation bath may incorporate, for example, di- or higher- valent cations (e.g. Ca²⁺) which serve to effect the precipitation and also stabilise the protein layer by ion bridging.

In a further method, the polysaccharide is extruded into a coagulation both which incorporates protein (containing the cell adhesion protein) as the coagulant. The protein coagulant may for example be an enriched blood plasma (containing a cell adhesion protein). Once again we believe that there is preferential deposition of the cell adhesion protein at the surface of the substrate. This procedure is particularly effective when the polysaccharide is chitosan.

In an alternative method of producing the substrate, a surface layer of a cell adhesion protein may be applied to a preformed polysaccharide. Application of the protein layer may be effected, for example, in a coating bath containing a solution of protein or by a technique such as spraying.

Examples of polysaccharides which may be used for the substrate include alginates, chitosan, cationic starches, cationic derivatives of other hydrocolloids, low-methoxyl pectin, carrageenans, chondroitin sulphate, hyaluronic acid, carboxymethyl cellulose, carboxymethyl starch, carboxymethyl guar, cellulose sulphate, dextran sulphate, gellan, xanthan, and anionic derivatives of other hydrocolloids. We particularly prefer that the polysaccharide is comprised of an alginate material cross-linked with calcium ions as other di- or higher valent cation capable of cross-linking alginates.

Particularly preferred examples of cell growth substrates in accordance with the invention are in the form of fibres having a core (providing the basal layer) which consists of, or is rich in, the polysaccharide material and a surface at which the cell adhesion protein is provided.

Fibres in accordance with the invention may have a diameter of 10-1000µm, more preferably 40-150µm, even more preferably 40-100µm, and ideally 50-80µm. the fibres may be of any appropriate length.

Such fibres may be produced by spinning a dope comprised of a solution of the polysaccharide into a coagulation bath causing precipitation of the fibres. The dope may also contain dissolved cell adhesion protein which is to form the surface layer with the spinning technique being such that there is preferential initial precipitation of polysaccharide in the coagulation bath followed by later precipitation of the cell adhesion protein which thus forms a protein rich outer layer of the fibre (this layer being integral with the core). The dope for use in this process may for example comprise (based on the total weight of the polysaccharide and cell adhesion protein) 60-95% (preferably about 90%) by weight of the polysaccharide and 5-40% (preferably about 10%) by weight of the cell adhesion protein. The fibre produced by such a process may have a core comprised of 50-80% by weight of the polysaccharide and an outer layer comprised of 50-80% by weight of the cell adhesion protein and 20-50% by weight of the polysaccharide.

In an alternative spinning method, fibres may be formed by a co-axial extrusion technique in which a solution of the cell adhesion protein is extruded coaxially around a (separate) solution of the polysaccharide, both solutions being spun into the same coagulation bath, whereby a fibre having a polysaccharide core and a surface layer of the cell adhesion protein is formed.

In an alternative process of producing the fibres, a dope comprised of a solution of the polysaccharide (but not the cell adhesion protein) may be spun into a coagulation bath and the fibre thus formed is treated with the cell adhesion protein. This treatment may be effected, for example, by providing the cell adhesion protein in the coagulation bath so that the protein is adsorbed as a surface layer onto the basal polysaccharide layer. It is however more preferred that the cell adhesion protein is applied in a bath downstream of the coagulation bath. The conditions in the protein bath may be such as to ensure formation of a stabilised coating of the protein layer is obtained.

Furthermore, for all embodiments of fibre formation, the cell adhesion protein should be concentrated at the fibre surface. If the fibre is produced by co-spinning a solution of the polysaccharide and cell adhesion protein the combination of relative molecular size, hydrophilic/hydrophobic balance and relative stability can be used to cause preferential precipitation If the fibre is produced by a two-stage process then concentration of the protein at the surface may be achieved by the use of concentration of the polysaccharide and protein at each stage, first stage mixed polysaccharide and protein, second stage predominantly protein plus surface active agents and/or stabilisers.

Whichever method is used, the protein should be stabilised at the surface and, in fact, the lower the amount of protein the more important the stabilisation becomes. Stabilisation may be effected by ensuring that parts of the molecular chain of the protein are embedded in the bulk polysaccharide. In the case where the polysaccharide has been cross-linked by divalent cations, stabilisation of the protein may be by divalent cation bridges- When chitosan is used to form the core, carrier cation bridging will only occur within the protein species which will help to stabilise the protein at the surface.

More specific embodiments of producing fibres in accordance with the invention are described below.

In one such embodiment, a fibre is produced by ejecting an aqueous solution of sodium alginate through a spinneret into a coagulation both containing Ca²⁻ ions. The fibre is then passed through a fibronectin solution (or mixed protein solution) in a coating bath (downstream of the coagulation bath) which is at a pH that will give fibronectin a net positive charge causing it to be capable of interacting with the alginic acid. The fibronectin can be further bound to the alginate by passing the fibre through a coagulation/stabilisation bath at a pH that favours fibronectin to become negatively charged thus favouring divalent cation bridging so as to stabilise the fibronectin on the polysaccharide. The coagulation/stabilisation bath may contain agents that modify either directly the interaction of the fibre with cells (for example through the nature of a counterion, e.g. Zm, Ag, Mn, Ce) or indirectly by influencing the surrounding environment by diffusion of an active molecular species, such as growth factors, aggregating agents, chemoattractants, surfactants, etc.

As an alternative to applying the fibronectin in a coating bath, it is possible to apply a fibronectin coating by spraying a fibronectin solution onto the fibre. Spraying provides a means of thin coating (i.e, only several molecules thick) and also a method of coating that will potentially produce a fibrillar form of the coating if the conditions of shear etc. are set correctly. These conditions may also be adjusted to give orientation of the fibril formed in relation to the substrate.

In a further embodiment of fibre production, fibres may be found in a single stage process by spinning a dope containing dissolved sodium alginate and fibronection into a solution of calcium or other divalent ions (which provide the driving force for precipitation). The dope is formulated such that the fibronectin is preferentially precipitated at the surface of the fibre. The relative amounts of the calcium alginate to the fibronectin in the dope would preferably be of the order of at least 80 parts by weight alginate and at most 20 parts fibronectin.

In the process described in the preceding paragraph, the fibre would be produced under conditions that encourage the globular nature of the protein. This may be achieved by use of a pH or temperature (for the coagulation bath) that causes chains of the protein molecule to "roll-up" on themselves with a tendency to embed the ends of the chain in the fibre structure.

In an alternative fibre production the process, a polyelectrolyte such as chitosan would be mixed with the fibronectin solution and a fibre precipitated by spinning into a sodium hydroxide bath. The molecular weight of the chitosan would be chosen to encourage fibre formation.

As an alternative to the process described in the previous paragraph it is possible to spin a dope comprising a solution of chitosan (as the polysaccharide) to form a fibre which may subsequently be coated with fibronectin. This coating (of fibronectin) would be formed by charge interaction directly between the charged chitosan side chains and the amino acid groups of the fibronectin as well as by cationic bridging.

For all methods of fibre production, it may be appropriate to subject the spun fibres to stretching, washing, and/or drying operations. In the case where a (separate) surface treatment of the cell adhesion protein is applied after formation of the basal polysaccharide layer, it may be appropriate to effect stretching and/or washing prior to the treatment with the cell adhesion protein.

Whilst fibres are the preferred form of the cell growth substrate in accordance with the invention, other forms are possible. Examples include sheets and strips which may be produced by forming (by a knife over roll or transfer coat or slot dye method) a thin film of a solution of the polysaccharide which is then precipitated in a coagulation bath. As in the case of fibre formation, the solution may also incorporate the cell adhesion protein to be preferentially deposited on coagulation at the surface of the polysaccharide. Alternatively the solution to be precipitated in the coagulation bath need not include the cell adhesion protein which may then be applied subsequently to the sheet or strip by spraying with a solution of the protein. In this case, the nature of the coating is determined by the concentration of the protein in solution, the velocity, orifice, size and direction of spray relative to the surface. Judicious adjustment of these parameters should produce undenatured but aligned molecules of active protein. The surface layer of the cell adhesion protein may be applied to the sheet by spraying with a solution of the protein. By spraying at high concentration and flow rate through a small orifice, protein denaturation, fibril formation and alignment can be obtained and if this is directed in parallel to a surface then this alignment will be maintained in the surface coat obtained molecular alignment of the protein will then be reflected in the alignment of cellular species grown on the substrate.

Irrespective of the physical form (fibre, sheet etc) of the cell growth substrate of the invention and also irrespective of the manner in which the cell adhesion protein surface layer is incorporated therein, it is preferred that basal polysaccharide layer is formed by a spinning or extruding a solution of sodium alginate into a bath containing calcium ions. Preferred sodium alginate for use in such a technique have a Guluronic acid (G) content of at least 35% by weight and a Mannuronic acid (M) content of at most 65% by weight. Preferably the G-content is 35-70% by weight and the M-content is 65-30% by weight. M preferably also the sodium alginate has a viscosity for a 1% solution (in water) of the sodium alginate of 30-300 cP, more preferably 40-100 cP. The alginate solution to be spun or extruded into the coagulation bath should generally have a total dissolved solids content of less than 10% by weight, more preferably in the range 5-7%. The amount of the cation (e.g. calcium) present in the coagulation bath (to effect precipitation of the alginate) is preferably less than 1% by weight.

For products in accordance with the invention produced by coagulation of a solution of an alginate, it is possible for the alginate solution (to be coagulated) to contain at least one additional polysaccharide to modify the properties of the alginate. The additional polysaccharide may, for example, be one having COO- groups along the polysaccharide chains, for example pectin, carboxymethyl cellulose N-, O-carboxymethyl chitosan, carrageenan, xanthan or gellan. Alternatively or additionally the polysaccharide to the coagulated with the alginate may be one having SO₄²⁻ groups provided along the polysaccharide chain, e.g. chondroitin sulphate, dermatan sulphate, heparan sulphate or heparan. Uncharged polysaccharides may be used in conjunction with the alginate, e.g. acemannan. The additional polysaccharide may be one which improves the water absorbency of the alginate. Further disclosure of products obtained by coagulation of an alginate solution containing at least one other polysaccharide are given in WO-A-9610106 (Innovative Technologies Ltd), the disclosure of which is incorporated herein by reference.

For all cell growth substrates in accordance with the invention, the surface layer of the cell adhesion protein may be continuous or discontinuous. Thus, for example, in the case of a fibre, the protein may be provided continuously along and around the fibre length or as periodic repeats (e.g. of predetermined length) along the fibre length and at least partially around the circumference of the fibre, or as "stripe" which does not extend completely around the circumference and which extends continuously or discontinuously along the fibre length. If the cell adhesion protein layer is discontinuous, parts of the surface of the cell growth substrate may (when used for cell growth) be positively interactive ("talking") and other parts passive ("silent") and other parts negatively interactive ("discouraging"). In cellular terms, this means that a positive surface encourages cell adhesion spreading, motility and growth whereas a passive surface ("silent") may have a low level of interaction

Cell growth substrates in accordance with the invention may be used in a number of forms for various cell growth applications. Purely by way of example, substrates in the form of fibres may be formed into a structure, e.g. random matrices (e.g. non-woven felts and fleeces), orientated matrices (fibres having some relative alignment), knitted structures (e.g. knitted cloths), braided structures (e.g. braided thread), bundled structures, and carded slivers. One preferred structure comprises fibres in accordance with invention laid in parallel or randomly to each other and for preference bonded to a supporting layer, e.g. a polyurethane film. This supporting layer may be adhesively coated.

A further possibility is for fibres to be arranged in an amorphous gel.

A further possibility relates to fibres produced with a polysaccharide (e.g. alginate) cross-linked by a di- or higher-valent cation (e.g. calcium). Such fibres may (using the techniques disclosed in WO-A-9613285 (Innovative Technologies Ltd) be admixed with an aqueous solution of a hydrogel precursor material whereby the cations from the fibres cross-link the precursor material resulting in the formation of a hydrogel in which the molecules of the hydrogel precursor are cross-linked by the di- or higher-valent cations donated by the fibres. The admixture may incorporate a plasticiser. Subsequently water may be removed from the hydrogel so as to provide a dehydrated form thereof containing the fibres as reinforcement. Such a product is eminently suitable for use on wound healing during which fibres will become exposed at the surface of the product to provide a substrate for cell growth. The hydrogel precursor may for example be sodium alginate and the plasticiser may for example be glycerol, polyethylene glycol, sorbitol or a PEO/PPO polymer.

Cell growth substrates in the form of strips or sheets may for example be rolled into tubes or other three dimensional structures.

As indicated above, cell growth substrates in accordance with the invention may be used in a range of cell growth applications. If cell alignment on the surface of the substrate is important then this may be imposed by the nature of the cell and its relationship to its surface. For example, cell alignment may be determined by the size of a fibre on which the cell is grown. If cell-long alignment either across or parallel to a particular axis of the substrate is required then this can be accomplished by either exposure of the surface to flow which will produce a wall shear stress parallel to the desired orientation or to axial strain which would tend to cause the cells to lie across the axis of stress and therefore across the axis of the surface.

A number of specific (but non-limiting) example of uses of cell growth substrates in accordance with the invention will now be given.

### Wound Therapy

The substrates may be used in wound therapy. For this purpose, a cell-growth substrate (in accordance with the invention) in the form of a flat sheet or film may be preferred. The film or sheet material may incorporate an agent to be delivered to the wound.

Alternatively, parallel or random arrays of fibres with or without seeded cells may be placed on the wound either individually, in a bundled or fixed to a support which may be adhesively coated. An example of a suitable support is polymeric film material particularly a breathable film (e.g. high MVTR film). The film may be one having an MVTR when in contact with liquid water which is at least twice that when in contact with moisture vapour (but not liquid water). For example, the MVTR in contact with water vapour only may be 3000-5000 g m⁻² 24hr⁻¹ (as measured by ASTM E96B) and an MVTR in the presence of liquid water (as measured by ASTM E96BW) of 8000 to 10000 g m⁻² 24hr⁻¹. The support may have apparatus to allow exudate transfer. Whether or not a support is used, the fibres applied to the wound may incorporate growth factors for delivery to surface cells or incorporate agents that will influence the surrounding environment, e.g. bactericides etc. Mixtures of fibres may be applied to the wound, e.g. any two of (i) fibres seeded with cells, (ii) unseeded fibres, and (iii) fibres containing an agent to be delivered to the wound.

### Cultured Epidermal and Dermal Substitutes

Cell growth substrates in accordance with the invention may be cultured with single layers of epidermal keratinocytes or dermal fibrobalasts (either of which may be of autologous or heterologous origin.) The substrate (with cultured cells) may be used alone or in combination with similarly cultured substrates. These substrates and cells may be used for the treatment of partial thickness wounds, e.g. donor sites and for treatment of ulcers.

### Tissue Augmentation/Repair

Cell growth substrates in the form of continuous fibres can be positioned in relation to a damaged organ or structure. They may be placed either singularly or in bundles during invasive or non-invasive therapy.

Alternatively, cell growth substrates in the form of fibres may be provided as an injectable suspension. The suspension may be introduced into the body along a catheter guide system or the fibres may be formed at the site. As an alternative, it is possible to formulate two solutions one containing the polysaccharide, the other coagulant therefore with at least one of the solutions containing cell adhesion protein and to apply these solutions to a patient under conditions such that fibre formation occurs *in situ,* the fibre formed possibly being continuous.

### Orthopaedic

Cell growth substrates in the form of fibres may be aligned parallel to tendons and seeded *in situ* with appropriate cells, chondrocytes, etc. Alternatively, fibres plus cell may be cultured in a laboratory and then delivered to the patient. For both embodiments, the fibres may contain, or be associated with fibres constructed with hyaluronic acid or other cartilage-derived substances.

### Vascular Graft

Cell growth substrates in the form of fibres may be knitted, woven or spun into tubes to encourage cell growth to form a blood conduit.

### Nerve Regeneration

Damaged nerves can be repaired using fibres to link the two (separated) ends of the nerve thus providing a path along which the new nerve can grow.

### Drug delivery

Cell growth substrates may incorporate active molecules located in the polysaccharide layer. These agents may be used to influence the fibre incorporation into the tissue. Alternatively the agent may provide a drug reservoir for the purposes topical or systemic therapy.

For all of the above embodiments of the invention, the cell adhesion protein may be replaced by a blood plasma component.

The invention is further illustrated with reference to the following Examples and the Figures of the accompanying drawings which show the results of the Examples.

For the Examples, the following procedures were used.

### Cell Culture

L929 mouse fibroblast cells for use in an experiment were grown to confluence and then released from the tissue culture dishes by washing with Hepes Saline, followed by treatment with 0.25% trypsin solution. The resulting supernatant was centrifuged and the pellet of cells re-suspended in Dulbecco's modified Eagle's Medium [containing 10% Foetal calf serum, 5% Penicillin/Streptomyocin, 1% ITS (Insulin transferrin selenite)]. If being sub-cultured, then the cells were plated out on tissue culture plates at a 1:5 dilution.

15mg of each fibre type to be tested were weighed out and placed in each well of a 12 well tissue culture dish. In all experiments the fibres were washed in serum containing media for a period of 24 hours. The experimental controls were cells plated on tissue culture plastic. Cells used for fibre testing were plated out at a density of 80,000 cells per well. All experiments were terminated up to a 72 hour timepoint.

### Fixation and Staining

The cells and fibres were washed twice in Phosphate Buffered Saline (PBS) and then fixed using formalin solution (10% neutral buffered) for 10 minutes. The fixative was removed and the cells and fibres washed twice more in PBS. The cells were then stained with Geimsa for 10 minutes, followed by 3, five minute washes in PBS. The cells were then viewed using a Nikon Diaphot microscope and images captured using a JVC DV1 digital camcorder. The images were then downloaded to an Apple Macintosh Power PC Performa 6400/200 and analysis performed using the public domain program NIH image. For the scanning electron microscopy, fixed samples were dehydrated in 100% ethanol for a period of 2 hours. The samples were then sputter coated using a Denton Vacuum desk 1. The samples were mounted on a stub and viewed using a Hitachi S-510 scanning electron microscope. Images are captured using the JVC camera and analysed on the Macintosh computer using NIH image.

### Preparation of Enriched Bovine Blood Plasma

Bovine blood was taken and mixed in a 9:1 ratio with a 4%w/w aqueous solution of trisodium citrate (Sigma Chemicals) as an anti-coagulant. The mixture was then centrifuged at 1000rpm for 10 minutes, after which time the supernatant plasma was pipetted off, frozen at -15 to 20 °C and then thawed under refrigeration at 4°C. This caused the globular protein content of the plasma to remain precipitated and become concentrated by sedimentation at the bottom of the storage vessel. The supernatant from the refrigerated plasma was removed and the remaining fraction when thawed formed a plasma further enriched in globular protein concentration which may be further enriched by another freeze thaw cycle. The plasma fraction thus isolated was used in some of the experiments outlined in the Examples below.

### Example 1 (Comparative)

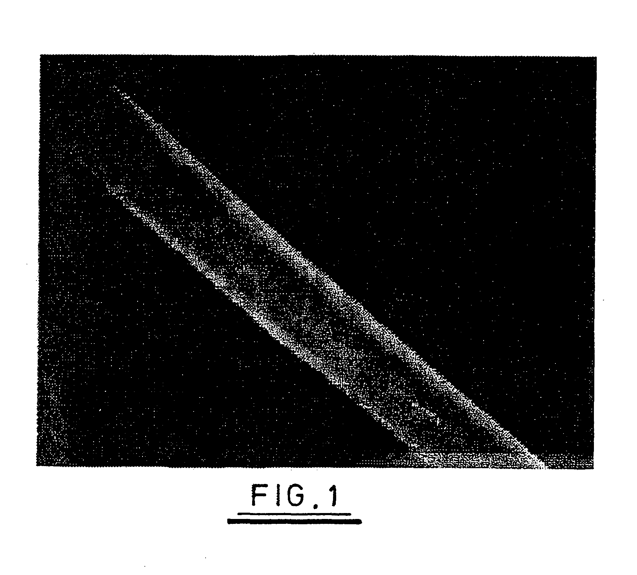

Calcium alginate fibres were produced by ejection at 12m/min of a 5.5%w/w aqueous solution of sodium alginate (ex Pronova Biopolymer, having a guluronic acid content of 70%) through a spinneret having 40,000 holes each of 70µ diameter into a coagulation bath of calcium chloride dihydrate (1.5%w/w) and the resulting fibres were washed in acetone and dried. The fibres were observed under a scanning electron microscope (Hitachi model S510) and were found to be about 10-20µ diameter smooth, cylindrical with few outstanding surface topographical features (see Figure 1). The cell adhesion properties of the fibres were then assessed using L929 mouse fibroblast cells according to the method outlined above. No cell attachment to the fibres was observed within 2 hours during which time the fibres formed a gel and then disintegrated.

### Example 2

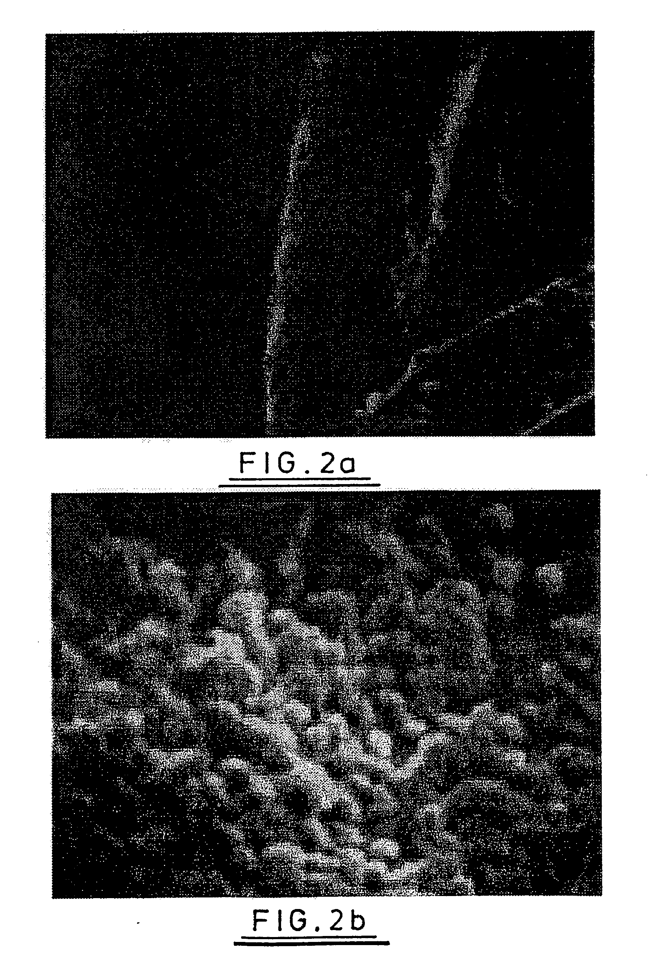

A mixture of 5.5%w/w aqueous solution of sodium alginate (as in Example 1) with bovine blood plasma was prepared by mixing the components in a ratio of 3:2. This mixture was then used to produce fibres in the laboratory by ejection from a I ml insulin syringe through a needle of 35µ outside diameter into a coagulation bath of calcium chloride dihydrate (1.5%w/w) and the resulting fibres were washed in acetone and dried. Fibres were observed under the scanning electron microscope (see Figure 2a). The cell adhesion properties of the fibres were then assessed using L929 mouse fibroblast cells according to the method outlined above. Within 48 hours cells had grown to confluence on the fibres (see Figure 2b), a considerable improvement of the result observed in Example 1.

### Example 3 (Comparative)

A 3% w/w of chitosan, having a degree of de-acetylation >70% (available from Nigerian Fisheries), in 2% aqueous glacial acetic acid was prepared.

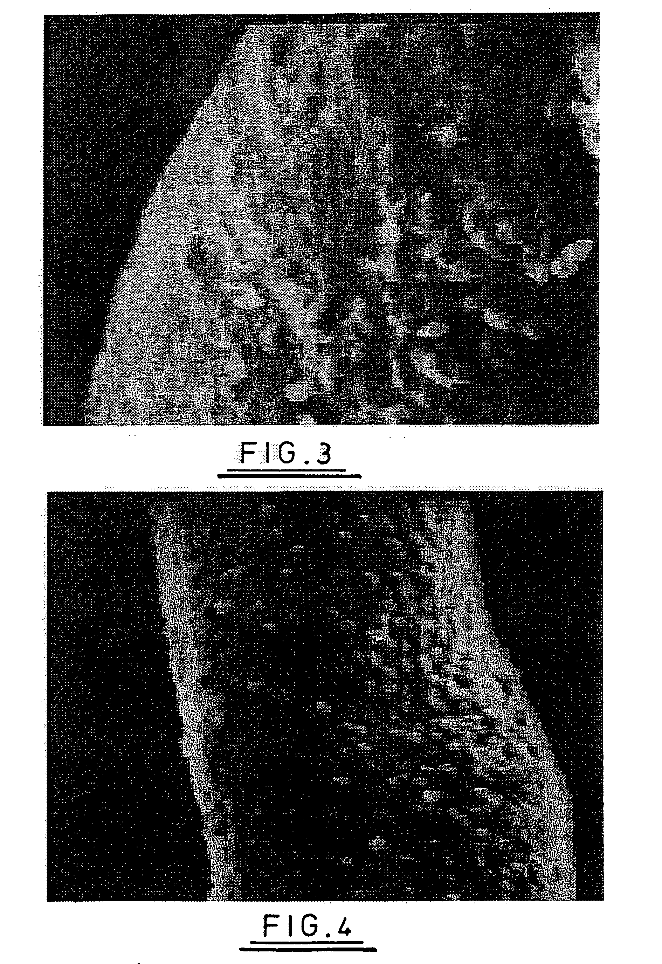

Chitosan fibres were made in the laboratory by ejecting the chitosan solution from a 1ml insulin syringe through a needle of 35µ outside diameter into a coagulation bath of sodium hydroxide (5%w/w) and the resulting fibres were dried. The fibres were observed under the scanning electron microscope, the fibres were found to have a diameter of 40-100µ and to be smooth, cylindrical with few outstanding surface topographical features. The cell adhesion properties of the fibres were then assessed using L929 mouse fibroblast cells according to the method outlined above. After 48 hours, a number of cells were found to adhere to the fibres but no evidence for cell elongation and alignment was apparent (see Figure 3).

### Example 4

Chitosan fibres were made in the laboratory by ejecting a solution of chitosan (as specified in Example 3) from a 1ml insulin syringe through a needle of 35µ outside diameter into a coagulation bath of enriched bovine blood plasma (isolated as described above) and the resulting fibres were washed in acetone and dried. The cell adhesion properties of the fibres were then assessed using L929 mouse fibroblast cells according to the method outlined above. Within 48 hours cells h ad grown to confluence (as seen from Figure 4) on the fibres, a far higher degree of cell attachment than that observed for fibres coagulated in sodium hydroxide (compare Example 3).

## Claims

1. A substrate for cell growth comprising a polysaccharide and a cell adhesion protein provided at the surface of the substrate and associated therewith by hydrogen bonding, van der Waals forces or physical entrapment.

2. A substrate as claimed in claim 1 comprising a polysaccharide basal layer having a surface layer incorporating the cell adhesion protein.

3. A substrate as claimed in claim 2 wherein the polysaccharide basal layer has a thickness greater than 60% of the combined thickness of the polysaccharide basal layer and cell adhesion protein layer.

4. A substrate as claimed in claim 3 wherein the polysaccharide basal layer has a thickness greater than 80% of the combined thickness of the polysaccharide basal layer and cell adhesion protein layer.

5. A substrate as claimed in any one of claims 2 to 4 wherein the cell adhesion protein layer is integral with the polysaccharide basal layer.

6. A substrate as claimed in claim 5 wherein the layer of the cell adhesion protein has a thickness of 1-20µm.

7. A substrate as claimed in any one of claims 2 to 5 wherein the layer of the cell adhesion protein is a surface adsorbed layer.

8. A substrate as claimed in claim 7 wherein the layer of the cell adhesion protein is 3-5 molecules deep.

9. A substrate as claimed in any one of claims 2 to 8 wherein the polysaccharide basal layer comprises at least 80% by weight of polysaccharide.

10. A substrate as claimed in claim 9 wherein the polysaccharide basal layer comprises at least 90% by weight of polysaccharide.

11. A substrate as claimed in any one of claims 2 to 10 wherein the surface layer of the cell adhesion protein comprises at least 80% by weight of cell adhesion protein.

12. A substrate as claimed in claim 11 wherein the surface layer of the cell adhesion protein comprises at least 90% by weight of cell adhesion protein.

13. A substrate as claimed in claim 12 wherein the surface layer of cell adhesion protein comprises 95 to 100% by weight of cell adhesion protein.

14. A substrate as claimed in any one of claims 2 to 13 wherein the cell adhesion protein layer is a discontinuous layer.

15. A substrate as claimed in any one of claims 2 to 14 wherein the polysaccharide layer incorporates an active agent selected from a drug, growth factor or chemotactic agent.

16. A substrate as claimed in claim 15 wherein the active agent is encapsulated.

17. A substrate as claimed in any one of claims 1 to 16 wherein the polysaccharide is selected from alginates, chitosan, cationic starches, cationic derivatives of other hydrocolloids, low-methoxyl pectin, carrageenans, chondroitin sulphate, hyaluronic acid, carboxymethyl cellulose, carboxymethyl starch, carboxymethyl guar, cellulose sulphate, dextran sulphate, gellan, xanthan, and anionic derivatives of other hydrocolloids.

18. A substrate as claimed in claim 17 wherein the polysaccharide is an alginate.

19. A substrate as claimed in claim 18 wherein the alginate has a Guluronic acid (G) content of at least 35% by weight and Mannuronic acid (M) content of at most 65% by weight.

20. A substrate as claimed in claim 19 wherein the polysaccharide as a G content of 35-70% by weight and an M content of 65-30% by weight.

21. A substrate as claimed in any one of claims 18 to 20 wherein the alginate is cross-linked with divalent cations.

22. A substrate as claimed in claim 21 wherein the divalent cations are calcium ions.

23. A substrate as claimed in claim 22 wherein the cell adhesion protein is stabilised by calcium ion bridges.

24. A substrate as claimed in claim 17 wherein the polysaccharide is chitosan.

25. A substrate as claimed in claim 24 wherein the chitosan comprises at least 70% de-acetylated chitin.

26. A substrate as claimed in any one of claims 1 to 25 wherein the cell adhesion protein is present in blood plasma.

27. A substrate as claimed in any one of claims 1 to 26 wherein the cell adhesion protein incorporates the RGD binding site.

28. A substrate as claimed in claim 27 wherein the cell adhesion protein is fibronectin, vitronectin or von Willebrand protein.

29. A substrate as claimed in claim 28 wherein the cell adhesion protein is fibronectin.

30. A substrate for cell growth comprising a polysaccharide and a blood plasma component provided at the surface of the substrate and non-covajently associated therewith by hydrogen bonding, van der Waals forces or physical entrapment.

31. A substrate as claimed in any one of claims 1 to 30 in the form of a fibre.

32. A substrate as claimed in claim 31 wherein the fibre has a diameter of 10-1000µm.

33. A substrate as claimed in claim 32 wherein the fibre has a diameter of 40-150µm.

34. A substrate as claimed in claim 33 wherein the fibre has a diameter of 40-100µm.

35. A substrate as claimed in claim 34 wherein the fibre has a diameter of 50-80µm.

36. A substrate as claimed in any one of claims 1 to 30 which is in the form of a sheet or film.

37. A substrate as claimed in claim 36 having a thickness of 2-2000µm.

38. A substrate as claimed in claim 37 having a thickness of 10-100µm.

39. A substrate as claimed in claim 37 having a thickness of 200-1000µm

40. A substrate as claimed in claim 37 having a thickness of 500-2000µm.

41. An assembly of fibres as claimed in any one of claims 31 to 35.

42. An assembly as claimed in claim 41 in the form of a random matrix, orientated matrix with fibres having some relative alignment, a knitted structure, a braided structure, a bundled structure or a carded sliver.

43. An assembly comprising a plurality of fibres as claimed in any one of claims 31 or 35 wherein the fibres are arranged in parallel to each other.

44. An assembly comprising a plurality of fibres as claimed in any one of claims 31 or 34 wherein the fibres are arranged randomly.

45. An assembly as claimed in claim 43 or 44 wherein the fibres are provided on a support in the form of a sheet or film.

46. An assembly as claimed in claim 45 wherein the fibres are provided on a high Moisture Vapour Transmission Rate film.

47. An assembly as claimed in claim 41 wherein said fibres are provided in a matrix of an amorphous gel.

48. A method of producing a cell growth substrate comprising extruding a solution containing dissolved polysaccharide and cell adhesion protein, the polysaccharides being present in an amount greater than the cell adhesion protein, into a coagulation bath such that there is precipitated a substrate comprised of a basal layer or a polysaccharide and a surface layer of cell adhesion protein associated therewith by hydrogen bonding, van der Waals forces or physical entrapment.

49. A method as claimed in claim 48 wherein the substrate is precipitated in a bath containing divalent cations.

50. As method as claimed in claim 49 wherein the divalent cations are calcium ions.

51. A method as claimed in any one of claims 48 to 50 wherein the solution to be extruded comprises, based on the total weight of the polysaccharide and cell adhesion protein, 60-99% by weight of the polysaccharide and 1-40% by weight of the cell adhesion protein.

52. A method as claimed in any one of claims 48 to 51 wherein the dissolved polysaccharide is sodium alginate.

53. A method as claimed in claim 52 wherein the sodium alginate has a G-content of 35-70% by weight and an M-content of 65-35% by weight.

54. A method as claimed in claim 52 or 53 wherein the sodium alginate has a viscosity for a 1% solution in water of 30-300 cP.

55. A method as claimed in claim 54 wherein the sodium alginate has a viscosity of a 1% solution in water of 40-100 cP.

56. A method of producing a cell growth substrate comprising applying to the surface of a preformed layer of a polysaccharide a surface layer of a cell adhesion protein or blood plasma component such that said cell adhesion protein or blood plasma component is associated with the polysaccharide by hydrogen bonding, van der Waals forces or physical entrapment.

57. A method as claimed in claim 56 wherein the method of application is by immersion of the polysaccharide layer in a coating bath containing the cell adhesion protein or blood plasma component.

58. A method as claimed in claim 56 wherein the polysaccharide is precipitated into a coagulation bath containing the cell adhesion protein.

59. A method as claimed in claim 56 wherein the method of application is by spraying.

60. A method as claimed in any one of claims 56 to 59 effected with stabilisation of the protein layer.

61. A method of *in vitro* cell culture comprising effecting growth of cells on a substrate as claimed in any one of claims 1 to 40 or an assembly as claimed in any one of claims 41 to 47.

62. A substrate as claimed in any one of claims 1 to 40 or an assembly as claimed in any one of claims 41 to 47 for use in therapy.

## Patentansprüche

1. Substrat für Zellwachstum, das ein Polysaccharid und ein Zelladhäsionsprotein aufweist, das an der Substratoberfläche bereitgestellt wird und durch Wasserstoffbindung, Van-der-Waals-Kräfte oder physikalischen Einschluß damit verbunden ist.

2. Substrat nach Anspruch 1, das eine Polysaccharid-Basalschicht mit einer Oberflächenschicht aufweist, die das Zellaääsionsprotein enthält.

3. Substrat nach Anspruch 2, wobei die Polysaccharid-Basalschicht eine Dicke aufweist, die größer ist als 60% der kombinierten Dicke der Polysaccharid-Basalschicht und der Zelladhäsionsproteinschicht.

4. Substrat nach Anspruch 3, wobei die Polysacharid-Basalschicht eine Dicke aufweist, die größer ist als 80% der kombinierten Dicke der Polysaccharid-Basalschicht und der Zelladhäsionsproteinschicht.

5. Substrat nach einem der Ansprüche 2 bis 4, wobei die Zelladhäsionsproteinschicht mit der Polysaccharid-Basalschicht integriert ist.

6. Substrat nach Anspruch 5, wobei die Schicht aus dem Zelladhäsionsprotein eine Dicke von 1-20 µm hat.

7. Substrat nach einem der Ansprüche 2 bis 5, wobei die Zelladhäsionsproteinschicht eine adsorbierte Oberflächenschicht ist.

8. Substrat nach Anspruch 7, wobei die Zelladhäsionsprotemschiehl 3 - 5 Moleküle tief ist.

9. Substrat nach einem der Anspruche 2 bis 8, wobei die Polysaccharid-Basalschicht mindestens 80 Gew-% Polysaccharid aufweist.

10. Substrat nach Anspruch 9, wobei die Polysaccharid-Basalschicht mindestens 90 Gew.-% Polysaccharid aufweist.

11. Substrat nach einem der Ansprüche 2 bis 10, wobei die Oberflächenschicht aus dem Zelladhäsionsprotein mindestens 80 Gew.-% Zelladhäsionsprotein aufweist.

12. Substrat nach Anspruch 11, wobei die Oberflächenschicht aus dem Zelladhäsionsprotein mindestens 90 Gew.-% Zelladhäsionsprotein aufweist.

13. Substrat nach Anspruch 12, wobei die Oberflächenschicht aus Zelladhäsionsprotein 95 bis 100 Gew.-% Zelladhäsionsprotein aufweist.

14. Substrat nach einem der Ansprüche 2 bis 13, wobei die Zelladhäsionsproteinschicht eine diskontinuierliche Schicht ist.

15. Substrat nach einem der Anspruch 2 bis 14, wobei die Polysaccharidschicht einen Wirkstoff enthält, der unter einem Medikament, einem Wachstumsfaktor oder einem chemotaktischen Wirkstoff ausgewählt ist,

16. Substrat nach Anspruch 15, wobei der Wirkstoff gekapselt ist.

17. Substrat nach einem der Ansprüche 1 bis 1 wobei das Polysaccharid unter Alginaten, Chitosan, kationischen Stärken, kationischen Derivaten anderer Hydrokolloide, methoxylarmem Pektin, Carrageenanen, Chondroitinsulfat, Hyaluronsäure, Carboxymethylcellulose, Carboxymethylstärke, Carboxymethylguar, Cellulosesulfat, Dextransulfat, Gellan, Xanthan und anionischen Derivaten anderer Hydrokolloide ausgewählt ist.

18. 8. Substrat nach Anspruch 17, wobei das Polysaccharid ein Alginat ist.

19. Substrat nach Anspruch 18, wobei das Alginat einen Guluronsäuregehalt (G-Gehalt) von mindestens 35 Gew.-% und einen Mannuronsäuregehalt (M-Gehalt) von höchstens 65 Gew.-% aufweist.

20. Substrat nach Anspruch 19, wobei das Polysaccharid einen G-Gehalt von 35-70 Gew.-% und einen M-Gehalt von 65-30 Gew.-% aufweist.

21. Substrat nach einem der Ansprüche 18 bis 20, wobei das Alginat mit zweiwertigen Kationen vernetzt ist.

22. Substrat nach Anspruch 21, wobei die zweiwertigen Kationen Calciumionen sind.

23. Substrat nach Anspruch 22, wobei das Zelladhäsionsprotein durch Calciumionenbrücken stabilisiert wird.

24. Substrat nach Anspruch 17, wobei das Polysaccharid Chitosan ist.

25. Substrat nach Anspruch 24, wobei das Chitosan mindestens 70% desacetyliertes Chitin aufweist.

26. Substrat nach einem der Ansprüche 1 bis 25, wobei das Zelladhäsionsprotein in Blutplasma anwesend ist.

27. Substrat nach einem der Ansprüche 1 bis 26, wobei das Zelladhäsionsprotein die RGD-Bindiingsstelle enthält.

28. Substrat nach Anspruch 27, wobei das Zelladhäsionsprotein Fibronectin, Vitronectin oder von-Willebrand-Protein ist.

29. Substrat nach Anspruch 28, wobei das Zelladhäsionsprotein Fibronectin ist

30. Substrat für Zellwachstum, das ein Polysaccharid und eine Blutplasmakomponente aufweist, die an der Substratoberfläche bereitgestellt und durch Wasserstoffbindung, Vau-der-Waals-Kräfte oder physikalischen Einschluß nichtkovalent daran gebunden wird.

31. Substrat nach einem der Ansprüche 1 bis 30 in Form einer Faser.

32. Substrat nach Anspruch 31, wobei die Faser einen Durchmesser von 10 - 1000 µm aufweist.

33. Substrat nach Anspruch 32, wobei die Faser einen Durchmesser von 40 - 150 µm aufweist.

34. Sttbstrat nach Anspruch 33, wobei die Faser einen Durchmesser von 40 - 100 µm aufweist.

35. Substrat nach Anspruch 34, wobei die Faser einen Durchmesser von 50 - 80 µm aufweist.

36. Substrat nach einem der Ansprüche 1 bis 30 in Form einer Folie oder eines Films.

37. Substrat nach Anspruch 36 mit einer Dicke von 2 - 2000 µm.

38. Substrat nach Anspruch 37 mit einer Dicke von 10- 100 µm.

39. Substrat nach Anspruch 37 mit einer Dicke von 200 - 1000 µm.

40. Substrat nach Anspruch 37 mit einer Dicke von 500 - 2000 µm.

41. Anordnung von Fasern nach einem der Ansprüche 31 bis 35.

42. Anordnung nach Anspruch 41 in Form einer zufälligen Matrix, einer orientierten Matrix mit Fasern, die eine bestimmte relative Ausrichtung aufweisen, einer gewirkten Struktur, einer geflochtenen Struktur, eine gebündelten Struktur oder eines Krempelbands.

43. Anordnung, die eine Vielzahl von Fasern nach einem der Ansprüche 31 oder 35 aufweist, wobei die Fasern parallel zueinander angeordnet sind.

44. Anordnung, die eine Vielzahl von Fasern nach einem der Ansprüche 31 oder 34 aufweist, wobei die Fasern zufällig angeordnet sind.

45. Anordnung nach einem der Ansprüche 43 oder 44, wobei die Fasern auf einem Träger in Form einer Folie oder eines Films aufgebracht sind.

46. Anordnung nach Anspruch 45, wobei die Fasern auf einem Film mit hoher Wasserdampfdurchlässigkeit aufgebracht sind.

47. Anordnung nach Anspruch 41, wobei die Fasern in einer Matrix aus einem amorphen Gel untergebracht sind.

48. Verfahren zur Herstellung eines Zellwachstumssubstrats, wobei das Verfahren aufweist: Extrudieren einer Lösung, die gelöstes Polysaccharid und Zelladhäsionsprotein enthält, wobei das Polysaccharid in einem höheren Anteil als das Zelladhäsionsprotein anwesend ist, in ein Koagulationsbad, so daß dort ein Substrat abgeschieden wird, das aus einer Basalschicht aus einem Polysaccharid und einer Oberflächenschicht aus Zelladhäsionsprotein besteht, die durch Wasserstoffbindung, Van-der-Waals-Kräfte oder physikalischen Einschluß daran gebunden ist.

49. Verfahren nach Anspruch 48, wobei das Substrat in ein Bad abgeschieden wird, das zweiwertige Kationen enthält.

50. Verfahren nach Anspruch 49, wobei die zweiwertigen Kationen Calciumionen sind.

51. Verfahren nach einem der Anspräche 48 bis 50, wobei die zu extrudierende Lösung, bezogen auf das Gesamtgewicht des Polysaccharids und des Zelladhäsionsproteins, 60 - 99 Gew.-% Polysaccharid und 1 - 40 Gew.-% Zelladbäsionsprotein aufweist.

52. Verfahren nach einem der Anspruche 48 bis 51, wobei das gelöste Polysaccharid Natriumalginat ist.

53. Verfahren nach Anspruch 52, wobei das Natriumalginat einen G-Gehalt von 35 - 70 Gew.-% und einen M-Gehalt von 65 - 35 Gew.-% aufweist.

54. Verfahren nach Anspruch 52 oder 53, wobei das Natriumalginat eine Viskosität für eine l%-ige Lösung (in Wasser) von 30-300 cP aufweist.

55. Verfahren nach Anspruch 54, wobei das Natriumalginat eine Viskosität einer 1%-igen Lösung (in Wasser) von 40- 100 cP aufweist.

56. Verfahren zur Herstellung eines Zellwachstumssubstrats, wobei das Verfahren aufweist: Aufbringen einer Oberflächenschicht aus einem Zelladhäsionsprotein oder einer Blutplasmakomponente auf die Oberfläche einer vorgeformten Schicht aus einem Polysaccharid, so daß das Zelladhäsionsprotein oder die Blutplasmakomponente durch Wasserstoffbindung, Varr-der-Waals-Kräfte oder physikalischen Einschluß an das Polysaccharid gebunden wird.

57. Verfahren nach Anspruch 56, wobei das Auftragsverfahren durch Eintauchen des Polysaccharidschicht in ein Beschichtungsbad ausgeführt wird, welches das Zelladhäsionsprotein order die Blutplasmakomponente enthält.

58. Verfahren nach Anspruch 56, wobei das Polysaccharid in ein Koagulationsbad abgeschieden wird, welches das Zelladhäsionsprotein enthält.

59. Verfahren nach Anspruch 56, wobei das Auftragsverfahren durch Sprühen ausgeführt wird.

60. Verfahren nach einem der Ansprüche 56 bis 59, das mit Stabilisierung der Proteinschicht ausgeführt wird.

61. Verfahren für eine In-vitro-Zellkultur, das aufweist: Bewirken von Zellwachstum auf einem Substrat nach einem der Ansprüche 1 bis 40 oder einer Anordnung nach einem der Anspruche 41 bis 47.

62. Substrat nach einem der Ansprüche 1 bis 40 oder Anordnung nach einem der Ansprüche 41 bis 47 zur therapeutischen Anwendung.

## Revendications

1. Substrat pour croissance cellulaire comprenant un polysaccharide et une protéine d'adhésion cellulaire disposée à la surface du substrat et associée à celui-ci par une liaison hydrogène, des forces de van der Waals ou un piégeage phasique.

2. Substrat selon la revendication 1 comprenant une couche basale de polysaccharide, ayant une couche superficielle renfermant la protéine d'adhésion cellulaire.

3. Substrat selon la revendication 2 dans lequel la couche basale de polysaccharide a une épaisseur supérieure à 60% de l'épaisseur combinée de la couche basale de polysaccharide et de la couche de protéine d'adhésion cellulaire.

4. Substrat selon la revendication 3 dans lequel la couche basale de polysaccharide a une épaisseur supérieure à 80% de l'épaisseur combinée de la couche basale de polysaccharide et de la couche de protéide d'adhésion cellulaire.

5. Substrat selon l'une quelconque des revendications 2 à 4 dans lequel la couche de protéine d'adhésion cellulaire fait partie intégrante de la couche basale de polysaccharide.

6. Substrat selon la revendication 5 dans lequel la couche de la protéine d'adhésion, cellulaire a une épaisseur de 1.-20 µm.

7. Substrat selon l'une quelconque des revendications 2 à 5 dans lequel la couche de la protéine d'adhésion cellulaire est une couche adsorbée de surface.

8. Substrat selon la revendication 7 dans lequel la couche de la protéine d'adhésion cellulaire fait 3.5 molécules de profondeur.

9. Substrat selon l'une quelconque des revendications 2 à 8 dans lequel la couche basale de polysaccharide comprend au moins 80% en poids de polysaccharide.

10. Substrat selon la revendication 9 dans lequel la couche basale de polysaccharide comprend au moins 90% en poids de polysaccharide.

11. Substrat selon l'une quelconque des revendications 2 à 10 dans lequel la couche superficielle de la protéine d'adhésion cellulaire comprend au moins 80% en poids de protéine d'adhésion cellulaire.

12. Substrat selon la revendication 11 dans lequel la couche superficielle de la protéine d'adhésion cellulaire comprend au moins 90% en poids de protéine d'adhésion cellulaire.

13. Substrat selon la revendication 12 dans lequel la couche superficielle de protéine d'adhésion cellulaire comprend 95 à 100% en poids de protéine d'adhésion cellulaire.

14. Substrat selon l'une quelconque des revendications 2 à 13 dans lequel la couche de protéine d'adhésion cellulaire est une couche discontinue.

15. Substrat selon l'une quelconque des revendications 2 à 14 dans lequel la couche de polysaccharide renferme un agent actif choisi parmi un médicament, un facteur de croissance ou un agent chimiotactique.

16. Substrat selon la revendication 15 dans lequel l'agent actif est encapsulé.

17. Substrat selon l'une quelconque des revendications 1 à 16 dans lequel le polysaccharide est choisi parmi les alginates, le chitosane, les amidons cationiques, les dérivés cationiques d'autres hydrocolloïdes, la pectine à faible taux de méthoxyl, les carraghénanes, le chondroïtine sulfate, l'acide hyaluronique, la carboxymethylcellulose, le carboxyméthylamidon, le carboxyméthylguar, le sulfate de cellulose, le sulfate de dextran, le gellane, le xanthane, et les dérivés anioniques d'autres hydrocolloïdes.

18. Substrat selon la revendication 17 dans lequel le polysaccharide est un alginate.

19. Substrat selon la revendication 18 dans lequel l'alginate a une teneur en acide guluronique (G) d'au moins 35% en poids et une teneur en acide mannuronique (M) de 65% en poids au plus.

20. Substrat selon la revendication 19 dans lequel le polysaccharide a une teneur en G de 35-70 % en poids et une teneur en M de 65-30 % en poids.

21. Substrat selon l'une quelconque des revendications 18 à 20 dans lequel l'alginate est réticulé avec des cations divalents.

22. Substrat selon la revendication 21 dans lequel les cations divalents sont des ions calcium.

23. Substrat selon la revendication 22 dans lequel la protéine d'adhésion cellulaire est stabilisée par des ponts d'ions calcium.

24. Substrat selon la revendication 17 dans lequel le polysaccharide est le chitosane.

25. Substrat selon la revendication 24 dans lequel le chitosane comprend au moins 70% de chitine désacétylée.

26. Substrat selon l'une quelconque des revendications 1 à 25 dans lequel la protéine d'adhésion cellulaire est présente dans le plasma sanguin.

27. Substrat selon l'une quelconque des revendications 1 à 26 dans lequel la protéine d'adhésion cellulaire renferme le site de liaison RGD.

28. Substrat selon la revendication 27 dans lequel la protéine d'adhésion cellulaire est la fibronectine, la vitronectine ou la protéine de von Willebrand,

29. Substrat selon la revendication 28 dans lequel la protéine d'adhésion cellulaire est la fibronectine.

30. Substrat pour croissance cellulaire comprenant un polysaccharide et un composant du plasma sanguin disposé à la surface du substrat et associé de manière non covalente à celui-ci par une liaison hydrogène, des forces de van der Waals ou un piégeage physique.

31. Substrat selon l'une quelconque des revendication 1 à 30 sous la forme d'une fibre.

32. Substrat selon la revendication 31 dans lequel la fibre a un diamètre de 10-1000 µm.

33. Substrat selon la revendication 32 dans lequel la fibre a un diamètre de 40-150 µm.

34. Substrat selon la revendication 33 dans lequel la fibre a un diamètre de 40-100 µm.

35. Substrat selon la revendication 34 dans lequel la fibre a un diamètre de 50-80 µm.

36. Substrat selon l'une quelconque des revendications 1 à 30 qui est sous la forme d'une feuille ou d'un film.

37. Substrat selon la revendication 36 ayant une épaisseur de 2-2000 µm.

38. Substrat selon la revendication 37 ayant une épaisseur de 10-100 µm.

39. Substrat selon la revendication 37 ayant une épaisseur de 200-1000 µm.

40. Substrat selon la revendication 37 ayant une épaisseur de 500-2000 µm.

41. Assemblage de fibres selon l'une quelconque des revendications 31 à 35.

42. Assemblage selon la revendication 41 sous la forme d'une matrice aléatoire, d'une matrice orientée avec des fibres ayant un certain alignement relatif, d'une structure tricotée, d'une structure tressée, d'une structure en faisceaux ou d'un ruban cardé.

43. Assemblage comprenant une pluralité de fibres selon l'une quelconque des revendications 31 ou 35 dans lequel les fibres sont disposées parallèlement les unes aux autres.

44. Assemblage comprenant une pluralité de fibres selon l'une quelconque des revendications 31 ou 34 dans lequel les fibres sont disposées de manière aléatoire.

45. Assemblage selon la revendication 43 ou 44 dans lequel les fibres sont disposées sur un support sous la forme d'une feuille ou d'un film.

46. Assemblage selon la revendication 45 dans lequel les fibres sont disposées sur un film ayant un taux de transmission de la vapeur d'eau (MVTR) élevé.

47. Assemblage selon la revendication 41 dans lequel lesdites fibres sont disposées dans une matrice d'un gel amorphe.

48. Procédé de production d'un substrat pour croissance cellulaire comprenant l'extrusion d'une solution contenant un polysaccharide et une protéine d'adhésion cellulaire dissous, le polysaccharide étant présent en une quantité supérieure à la protéine d'adhésion cellulaire, dans un bain de coagulation de sorte qu'il y précipite un substrat composé d'une couche basale d'un polysaccharide et d'une couche superficielle de protéine d'adhésion cellulaire qui lui est associée par une liaison hydrogène, des forces de van der Waals ou un piégeage physique.

49. Procédé selon la revendication 48 dans lequel le substrat est précipité dans un bain contenant des cations divalents.

50. Procédé selon la revendication 49 dans lequel les cations divalents sont des ions calcium.

51. Procédé selon l'une quelconque des revendications 48 à 50 dans lequel la solution à extruder comprend, sur la base du poids total du polysaccharide et de la protéine d'adhésion cellulaire, 60-99% en poids du polysaccharide et 1-40% en poids de la protéine d'adhésion cellulaire.

52. Procédé selon l'une quelconque des revendications 48 à 51 dans lequel le polysaccharide dissous est l'alginate de sodium.

53. Procédé selon la revendication 52 dans lequel l'alginate de sodium a une teneur en G de 35-70% en poids et une teneur en M de 65-35% en poids,

54. Procédé selon la revendication 52 ou 53 dans lequel l'alginate de sodium a une viscosité pour une solution à 1% dans l'eau de 30-300 cP.

55. Procédé selon la revendication 54 dans lequel l'alginate de sodium a une viscosité d'une solution à 1% dans l'eau de 40-100 cP.

56. Procédé de production d'un substrat pour croissance cellulaire comprenant l'application sur la surface d'une couche préformée d'un polysaccharide, d'une couche superficielle d'une protéine d'adhésion cellulaire ou d'un composant du plasma sanguin de sorte que ladite protéine d'adhésion cellulaire ou ledit composant du plasma sanguin est associé(e) au polysaccharide par une liaison hydrogène, des forces de van der Waals ou un piégeage physique.

57. Procédé selon la revendication 56 dans lequel la méthode d'application est par immersion de la couche de polysaccharide dans un bain de revêtement contenant la protéine d'adhésion cellulaire ou le composant du plasma sanguin.

58. Procédé selon la revendication 56 dans lequel le polysaccharide est précipité dans un bain de coagulation contenant la protéine d'adhésion cellulaire.

59. Procédé selon la revendication 56 dans lequel la méthode d'application est par pulvérisation.

60. Procédé selon l'une quelconque des revendications 56 à 59 effectué avec stabilisation de la couche de protéine.

61. Procédé de culture cellulaire *in vitro* comprenant la croissance efficace de cellules sur un substrat selon l'une quelconque des revendications 1 à 40 ou un assemblage selon l'une quelconque des revendications 41. à 47.

62. Substrat selon l'une quelconque des revendications 1 à 40 ou assemblage selon l'une quelconque des revendications 41 à 47 pour une utilisation en thérapie.
